# EUROPEAN PATENT APPLICATION

(11) **EP 4 748 846 A1**
(43) Date of publication of application: **27.05.2026**
(21) Application number: 23945544.7
(22) Date of filing: 17.08.2023
(51) Int. Cl.: C07K 1/06, C07K 1/12

(54) **METHOD FOR SYNTHESIZING AMIDE AND/OR POLYPEPTIDE BY TAKING TRANSIENTLY PROTECTED AMINO ACID AS AMMONIA COMPONENT**

(30) Priority: 18.07.2023 CN 202310882096
(71) Applicant: GUANGZHOU MEDICAL UNIVERSITY, Guangzhou, Guangdong 511436 (CN)
(72) Inventor: ZHAO, Junfeng, Guangzhou, Guangdong 511436 (CN); WANG, Penghui, Guangzhou, Guangdong 511436 (CN); LIU, Tao, Guangzhou, Guangdong 511436 (CN)
(74) Representative: IP Sextant s.r.l.
(86) International application number: PCT/CN2023/113556
(87) International publication number: WO 2025/015659

(57) **Abstract**

Provided is a method for synthesizing amide and/or polypeptide by taking a transiently protected amino acid as an ammonia component. In the method, an allenone compound is taken as a condensation reagent, a silylating reagent is used for temporarily protecting a carboxyl group of an amino acid or peptide fragment of which amino and carboxyl groups are not protected, then an α-carbonyl alkenyl ester derivative of another molecular carboxylic acid, or amino acid, or a C-terminal carboxyl group of a polypeptide is used together with same to form an amide bond, and then the temporarily protected carboxyl group can be subjected to on-site deprotection under an acidic condition to obtain a target carboxylic acid product, so as to cyclically construct a new amide bond. The allenone condensation agent can effectively avoid racemization, and the "temporary protection" strategy enables the three-step reaction of "protection-condensation-deprotection" to be carried out in one pot, so that greatly excessive chemical reagents, time, manpower and energy consumption required when protecting groups are introduced and protecting groups are removed are avoided, thereby improving the step economy and atom economy of polypeptide synthesis.

## Description

### TECHNICAL FIELD

The present disclosure relates to the technical field of organic synthetic chemistry, and in particular relates to a method for synthesizing an amide and/or a polypeptide using a transient protected amino acid as an amine component.

### BACKGROUND

Polypeptides are compounds formed by dehydration condensation reaction between two or more amino acids, an irreplaceable raw material that constitutes enzymes, hormones, antibodies, nerve interstitium and other active substances in the human body, and the source of all life. Since there are basic α-amino and acidic α-carboxyl in each amino acid, the condensation between amino acids will produce a variety of mixtures in any combination. Therefore, the reactive groups that do not need to participate in the reaction must be protected before the condensation reaction, and then the condensation reaction is carried out to ensure the directional progress of the synthesis. Commonly used N-terminal protecting groups of amino acid include fluorenylmethoxycarbonyl (Fmoc), tert-butoxycarbonyl (Boc), benzyloxycarbonyl (Cbz), etc.; C-terminal protective groups of amino acid include methyl (Me), ethyl (Et), tert-butyl (tBu), etc. However, each step of the traditional peptide synthesis method requires the introduction and removal of protecting groups, which not only wastes a large amount of solvents and reagents, but also greatly increases the time cost of scientific researchers. With the development of chemistry and chemical biology, fast, efficient, economical, and environmental-friendly peptide synthesis methods have become a hot research topic.

In 2021, some scholars designed and developed a new allenone condensing agent for the synthesis of amide bonds and peptide bonds. This new condensing agent is easy to prepare, has good stability, small molecular weight, mild reaction conditions, and does not require any additives during use. More importantly, the condensing agent does not racemize when activating the peptide chain or during the ammonolysis process. The N-C polypeptide synthesis strategy can be adopted, which greatly improves the purity and yield of the product. However, this condensing agent still reacts with carboxyl-protected amino acids as the amine component during the polypeptide synthesis process. After the reaction is completed, the protected polypeptide is still obtained. To continue extending the peptide chain, deprotection post-processing is still required, resulting in tedious synthetic steps of the polypeptides. The steps are time-consuming, laborious, and costly.

### SUMMARY

The present disclosure aims to solve at least one of the above technical problems existing in the prior art. To this end, the object of the present disclosure is to provide a method for synthesizing an amide and/or a polypeptide using a transient protected amino acid as an amine component. In this method, by using an allenone compound as a condensing reagent, and a silylation reagent is used to temporarily protect the unprotected amino acid of amino and carboxyl or the carboxyl of a peptide segment first, and then after forming a peptide bond with another molecular amino acid or an α-carbonyl alkenyl ester derivative of the C-terminal carboxyl of the polypeptide, the temporarily protected carboxyl may be deprotected on site under an acidic condition to obtain a target carboxylic acid product to cyclicly build new peptide bonds. The condensing agent or the activating agent used when the polypeotides are synthesized with unprotected amino acids in the existing related art can only synthesize dipeptides. When tripeptides or polypeptides containing three or more than three amino acids are synthesized, severe racemization will be induced. This is because traditional condensing agents activate long-chain polypeptides. The allenone condensing agent used in the present disclosure can effectively avoid racemization, and this "temporary protection" strategy enables the three-step reaction of "protection-condensation-deprotection" to be carried out in one pot, merging the original three steps into one step, which avoids the large excess of chemical reagents, time, manpower and energy consumption required when introducing and removing protective groups, improves the step economy and atom economy of peptide synthesis, and greatly reduces the cost of peptide synthesis.

In order to achieve the above object, the technical solution adopted by the present disclosure is as follows:
In a first aspect of the present disclosure, provided is a method for preparing an amides and/or a polypeptide, including the following steps: 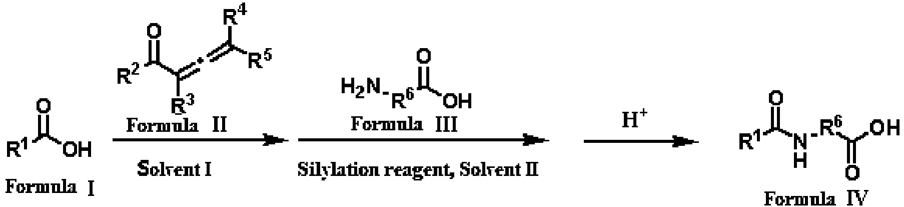
reacting a compound of formula I with a compound of formula II in a solvent I, and carrying out a nucleophilic substitution reaction with a compound of formula III and a silylation reagent in a solvent II, and then obtaining a compound of formula IV after acidification;
wherein R¹ includes alkyl, alkenyl, alkynyl, cycloalkyl, heterocycloalkyl, aryl, heteroaryl, an amino acid residual body lacking C-terminal carboxyl, an amino acid derivative residual body or a polypeptide fragment lacking C-terminal carboxyl; R² includes one of aryl, substituted aryl, heteroaryl, and substituted heteroaryl; R³, R⁴ and R⁵ each independently include one of H, C₁-C₁₈ hydrocarbonyl, substituted C₁-C₁₈ hydrocarbonyl, C₁-C₁₆ acyl, cyano, and C₁-C₁₆ hydrocarbonyl-carbonyl; R⁶ includes alkyl, cycloalkyl, heterocycloalkyl, aryl, heteroaryl, an amino acid residue or a polypeptide fragment.

In some embodiments of the present disclosure, in R², the substituted aryl or substituted heteroaryl includes aryl or heteroaryl which is substituted by at least one of halogen, C₁-C₆ alkyl, C₁-C₆ alkoxy, and nitro.

In some embodiments of the present disclosure, R² includes one of phenyl, 4-fluorophenyl, 4-chlorophenyl, 4-bromophenyl, 4-methylphenyl, 4-methoxyphenyl, 2-methyl phenyl, 2-methoxyphenyl, 3,5-dimethoxyphenyl, 3-nitrophenyl, 4-nitrophenyl, 2,4-dinitrophenyl, 3,5-dinitrophenyl, pentafluorophenyl, 4-trifluoromethylphenyl, 3,5-dichlorophenyl, 1-naphthyl, 2-naphthyl, furyl, thienyl, and pyridyl.

In some embodiments of the present disclosure, R³, R⁴ and R⁵ each independently include one of H, methyl, formyl, acetyl, propionyl, cyano, methoxycarbonyl, ethoxycarbonyl, tert-butoxycarbonyl, and carbobenzoxy.

In some embodiments of the present disclosure, the alkyl refers to a saturated hydrocarbyl having a specified number of carbon atoms. For example, C₁-C₁₈ alkyl refers to an alkyl group having 1 to 18 carbon atoms, such as preferably 1 to 6 carbon atoms. The alkyl group can be straight-chain or branched-chain. A representative branched-chain alkyl group has one, two or three branched chains. The alkyl group may also be optionally substituted with one or more substituents as defined herein. Specific examples of the alkyl include methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3dimethylbutyl, 2,3-dimethylbutyl, 2-ethylbutyl, 1,2-dimethylpropyl, neopentyl, hexyl, 2-methylpentyl, etc.

In some embodiments of the present disclosure, the heterocycloalkyl refers to a cyclic group that is fully saturated and can exist as a single ring, a bridged ring or a spiro ring. Unless otherwise indicated, the heterocycle is typically a 3 to 10 membered ring containing 1 to 3 heteroatoms (preferably 1 or 2 heteroatoms) independently selected from sulfur, oxygen and/or nitrogen, or the heterocycle is typically a 3 to 7 membered ring containing 1 to 3 heteroatoms (preferably 1 or 2 heteroatoms) independently selected from sulfur, oxygen and/or nitrogen. The heterocycloalkyl can be a 3 to 6 membered ring containing 1 or 2 heteroatoms independently selected from oxygen and nitrogen. Examples of 3-membered heterocycloalkyl include, but are not limited to, oxirane, ethylene sulfide, and aziranyl. Non-limiting examples of 4-membered heterocycloalkyl include, but are not limited to, acridinyl, oxetanyl, and thibutylcyclyl. Examples of 5-membered heterocycloalkyl include, but are not limited to, tetrahydrofuryl, tetrahydrothienyl, pyrrolidinyl, isoxazolidinyl, oxazolidinyl, isothiazolidinyl, thiazolidinyl, imidazolidinyl, and tetrahydropyrazolyl. Examples of 6-membered heterocycloalkyl include, but are not limited to, piperidyl, tetrahydropyranyl, tetrahydrothianyl, morpholinyl, piperazinyl, 1,4-thioxanyl, 1,4-dioxanyl, thiomorpholinyl, 1,3-dithianyl, 1,4-dithianyl. Examples of 7-membered heterocycloalkyl include, but are not limited to, azepanyl, oxeptanyl, and thiopanyl. The rest of the multi-membered heterocycles can be deduced in the same way.

In some embodiments of the present disclosure, the cycloalkyl refers to a carbon ring that is fully saturated and may exist in the form of a single ring, a bridged ring or a spiro ring. Unless otherwise indicated, the carbon ring is generally a 3 to 18 membered ring, or a 3 to 10 membered ring. Non-limiting examples of the cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, etc.

In some embodiments of the present disclosure, the aryl is selected from phenyl, o-tolyl, m-tolyl, p-tolyl, 2,4-xylyl, p-cumenyl, mesityl, 1-naphthyl, 2-naphthyl, 1-anthracenyl, 2-anthracenyl, 9-anthracenyl, 1-phenanthrenyl, 9-phenanthrenyl, 1-acenaphthyl, 2-acenaphthyl, 1-pyrenyl, 2-triphenylene, o-biphenyl, m-biphenyl, p-biphenyl, and terphenyl.

In some embodiments of the present disclosure, the heteroaryl is selected from heterocyclyl having 1 or 2 nitrogen atoms, oxygen atoms or sulfur atoms, and being preferably 5 to 10 member heterocyclyl. For example, triazolyl,3-oxadiazolyl, 2-furyl, 3-furyl, 2-thienyl, 3-thienyl, 1-pyrrolyl, 2-pyrrolyl, 3-pyrrolyl, 2-pyridyl, 3-pyridyl, 4-pyridyl, 2-pyrazinyl, 2-oxazolyl, 3-isoxazolyl, 2-thiazolyl, 3-isothiazolyl, 2-imidazolyl, 3-pyrazolyl, 2-quinolyl, 3-quinolyl, 4-quinolyl, 5-quinolyl, 6-quinolyl, 7-quinolyl, 8-quinolyl, 1-isoquinolyl, 2-quinoxalinyl, 2-benzofuranyl, 2-benzothienyl, N-indolyl, and N-carbazolyl can be listed.
In some embodiments of the present disclosure, in R¹, the amino acid residual body lacking C-terminal carboxyl or the amino acid derivative residual body lacking C-terminal carboxyl refers to the remaining part of the amino acid or amino acid derivative after the C-terminal carboxyl group is deleted; preferably, the amino acid residual body lacking C-terminal carboxyl includes at least one of an α-amino acid residual body lacking C-terminal carboxyl, a β-amino acid residual body lacking C-terminal carboxyl, or a γ-amino acid residual body lacking C-terminal carboxyl; preferably, the amino acid derivative residual body lacking C-terminal carboxyl includes an amino acid residual body lacking C-terminal carboxyl protected C-terminal amino, such as an amino acid residual body lacking C-terminal carboxyl with the C-terminal amino being protected by N-alkoxycarbonyl and/or N-acyl; preferably, α-amino acid is generally represented by 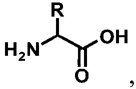 then in R¹, the amino acid residual body lacking C-terminal carboxyl is 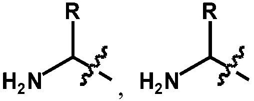 can be in R or S configuration; specifically, the amino acid residual body lacking C-terminal carboxyl is selected from 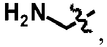 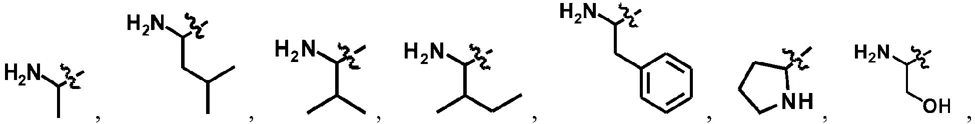 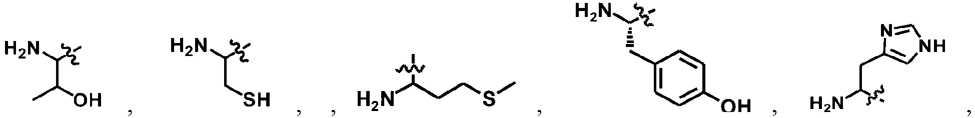 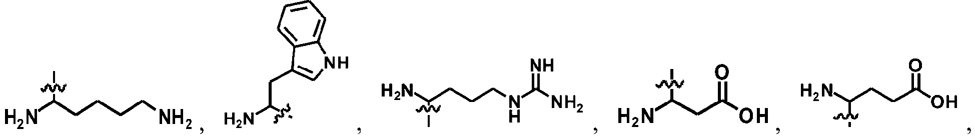 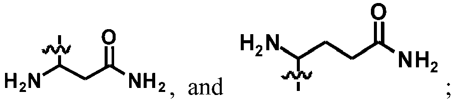 preferably, the amino acid derivative residual body lacking C-terminal carboxyl is selected from an amino acid residual body lacking C-terminal carboxyl in which the amino group at C-terminal is protected by N-alkoxycarbonyl and/or N-acyl, among the above-mentioned amino acid residual body lacking C-terminal carboxyl.

In some embodiments of the present disclosure, when the solvent II is different from the solvent I, the method for preparing an amide and/or a polypeptide further includes removing the solvent I after the reaction and then performing the nucleophilic substitution reaction.

In some embodiments of the present disclosure, the acidifying agent for acidifying includes organic acids and/or inorganic acids.

The term "acidifying agent" may mean any material that lowers the pH of a solution by being dissolved in water. In some embodiments, the acidifying agent may be an inorganic acid and/or an organic acid that can be dissolved in water to lower the pH of the solution to 5 or lower (such as, pH ≤4, pH≤3, pH≤2, pH≤1).

The inorganic acid may include hydrochloric acid, hydrobromic acid, hydroiodic acid, phosphoric acid, potassium dihydrogen phosphate, sodium dihydrogen phosphate, sulfonic acid, sulfamic acid, sulfuric acid, nitric acid, or any combination thereof. However, examples are not limited thereto. The organic acid may include citric acid, lactic acid, tartaric acid, fumaric acid, trifluoroacetic acid, phthalic acid, acetic acid, oxalic acid, malonic acid, adipic acid, phytic acid, succinic acid, glutaric acid, maleic acid, malic acid, mandelic acid, ascorbic acid, benzoic acid, methanesulfonic acid, p-toluenesulfonic acid, salicylic acid, capric acid, caproic acid, caprylic acid, lauric acid, arachidic acid, erucic acid, linoleic acid, linolenic acid, oleic acid, palmitic acid, myristic acid, edisilic acid, stearic acid or any combination thereof. However, examples are not limited thereto.

In a second aspect of the present disclosure, provided is another method for preparing an amide and/or a polypeptide, including the following steps: 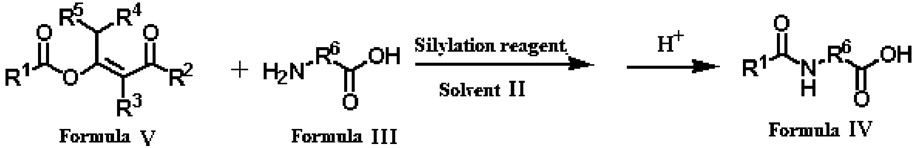
dissolving a compound of formula V, a compound of formula III, and a silylation reagent in a solvent II to perform a nucleophilic substitution reaction, and then preparing a compound of formula IV after acidification;
wherein R¹, R², R³, R⁴, R⁵ and R⁶ are defined as mentioned above.

In the present disclosure, since the compound of formula III (a compound with both amino and carboxyl) exists in the form of an intramolecular zwitterion under neutral conditions, it has poor solubility, the amino does not have nucleophilicity, and it is difficult to perform a condensation reaction. The use of the silylation reagent (a transient protection reagent) can temporarily protect the carboxyl in the compound of formula III to form a silicon ester compound, and at the same time free its amino, which can then undergo a nucleophilic substitution reaction with the compound of formula V to form a peptide bond to prepare a polypeptide compound of formula IV, and at the same time, the compound of formula VI is formed as a by-product. The reaction mechanism is as follows:

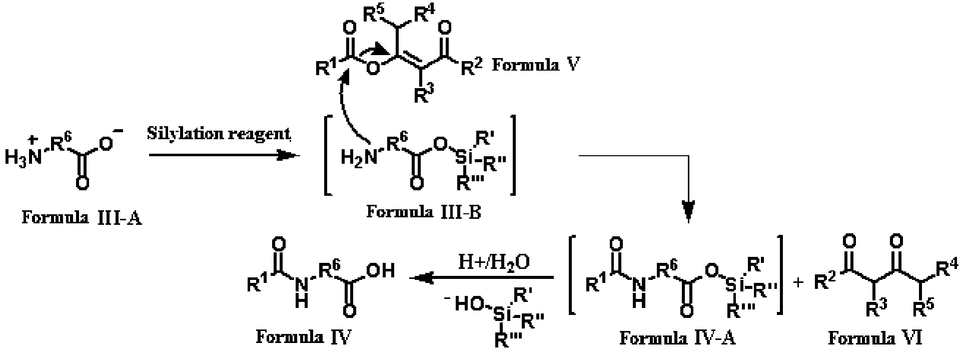

In some embodiments of the present disclosure, the silylation reagent includes at least one of N-trimethylsilylacetamide, N-methyl-N-trimethylsilylacetamide, N-trimethylsilylpyrrolidone, N-(tertbutyldimethylsilyl)-N-methyltrifluoroacetamide, N,O-bis(tert-butyldimethylsilyl)acetamide, N,O-bistrimethylsilylacetamide, N,O-bis(trimethylsilyl)trifluoroacetamide, N-methyl-N-(trimethylsilyl)trifluoroacetamide, hexamethyldisilylurea, trimethylsilimidazole, dimethyl dichlorosilane, trimethylchlorosilane, and tert-butyldimethylchlorosilane.

In some embodiments of the present disclosure, the compound of formula III is an organic compound having both amino and carboxyl. Preferably, the compound of formula III includes any one of amino acid compounds, polypeptide compounds with free amino and carboxyl, or other compounds with both amino and carboxyl. Further preferably, the compound of formula III includes any one of α-amino acid, β-amino acid, γ-amino acid or a polypeptide compound with unprotected amino and carboxyl. Still further preferably, the compound of formula III includes any one of glycine, alanine, leucine, isoleucine, proline, valine, phenylalanine, tyrosine, serine, threonine, cysteine acid, methionine, aspartic acid, glutamic acid, asparagine, glutamine, tryptophan, histidine, lysine, arginine, citrulline or derivative compounds of the above amino acids. It should be understood that the twentyone amino acids included in the compound of formula III can all be natural α-amino acids in the L-configuration and/or their corresponding α-amino acids in the D-configuration.

In some embodiments of the present disclosure, a molar ratio of the silylation reagent to the compound of formula III is (0.5-20): 1, preferably (1-15): 1, further preferably (2-10): 1.

In some embodiments of the present disclosure, the solvent II includes at least one of acetonitrile, diethyl ether, ethylene glycol dimethyl ether, ethylene glycol diethyl ether, dioxane, dimethyl sulfoxide, N,N-dimethylformamide, N,N-dimethylacetamide, N-methylpyrrolidone, toluene, m-xylene, dichloromethane, 1,2-dichloroethane or chloroform.

In some embodiments of the present disclosure, the temperature of the nucleophilic substitution reaction is -20°C to 150°C, preferably 0°C to 90°C, and further preferably 5°C to 50°C.

In some embodiments of the present disclosure, the nucleophilic substitution reaction time is at least about 10 minutes, preferably 10 min to 72 h.

In some embodiments of the present disclosure, the method for preparing an amide and/or a polypeptide includes the following steps: dissolving the compound of formula III and the silylation reagent in the solvent II, and then adding the compound of formula V to perform a nucleophilic substitution reaction to prepare the compound of formula IV.

In some embodiments of the present disclosure, the method for preparing a polypeptide includes the following steps: stirring the compound of formula III and the silylation reagent at 20°C to 50°C for 5 min to 24 hours and dissolving the same in the solvent II, and then adding the compound of formula V to perform a nucleophilic substitution reaction to prepare the compound of formula IV.

In some embodiments of the present disclosure, the method for preparing an amide and/or a polypeptide further includes the step of purifying the product after the nucleophilic substitution reaction. The purification includes at least one of extraction, recrystallization or column chromatography.

In some embodiments of the present disclosure, the preparation method of the compound of formula V includes the following steps: 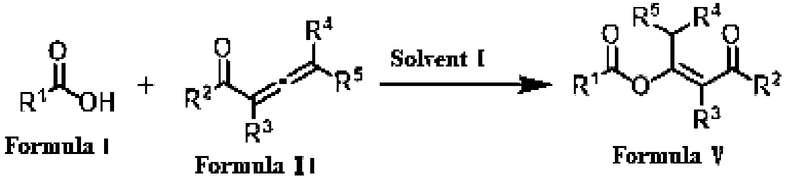
dissolving a compound of formula I and a compound of formula II in a solvent I to obtain a mixture, and reacting the mixture under stirring to prepare a compound of formula V;
wherein R¹, R², R³, R⁴ and R⁵ are defined as mentioned above.

In some embodiments of the present disclosure, the solvent I includes at least one of dichloromethane, chloroform, 1,2-dichloroethane, diethyl ether, toluene, acetonitrile, methanol or ethanol.

In some embodiments of the present disclosure, the compound of formula I is a carboxylic acid compound; preferably, the compound of formula I includes at least one of fatty acid, aromatic acid, heterocyclic acid, acetylenic acid, olefine acid, N-acylamino acid, N-alkoxycarbonyl amino acid or polypeptide carboxylic acid; further preferably, the compound of formula I includes at least one of N-benzyloxycarbonyl amino acid, N-tert-butoxycarbonyl amino acid, N-fluorenylmethoxycarbonyl amino acid, N-acetyl amino acid or polypeptide carboxylic acid.

In some embodiments of the present disclosure, a molar ratio of the compound of formula I to the compound of formula II is 1: (1-5), preferably 1: (1-4), further preferably 1: (1-2).

In some embodiments of the present disclosure, the temperature of the stirring reaction is -20°C to 90°C, preferably 0°C to 50°C.

In a third aspect of the present disclosure, provided is a method for preparing an amide and/or a polypeptide, including the following steps: 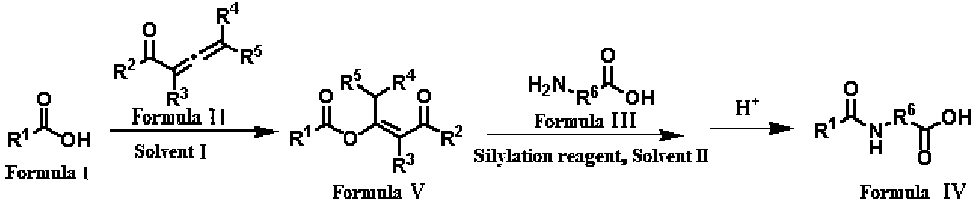
S1: dissolving a compound of formula I and a compound of formula II in a solvent I to obtain a mixture, and reacting the mixture under stirring to prepare the compound of formula V;
S2: dissolving the compound of formula V, the compound of formula III and a silylation reagent in a solvent II to perform a nucleophilic substitution reaction, and then preparing a compound of formula IV after acidification;
wherein R¹, R², R³, R⁴, R⁵ and R⁶ are defined as mentioned above.

In a fourth aspect of the present disclosure, provided is a use of the method for preparing an amide and/or a polypeptide in preparing amides and/or polypeptides.

The beneficial effects of the present disclosure are as follows:
in the method for preparing a polypeptide of the present disclosure, by using an allenone compound as a condensing reagent and low-cost unprotected amino acids as a raw material, a silylation reagent is used to temporarily protect the amino acids to perform the condensation reaction, so that "protection-condensation-deprotection" can be achieved in one pot. The three-step reactions are merged into one step. After the construction of the peptide bond is achieved, the target polypeptide carboxylic acid can be obtained by simple acidification treatment, which can be directly used for the condensation of the next amino acid. Moreover, based on the characteristics of the allenone condensation agent, this method can also completely avoid the formation of racemic by-products, ensuring the yield and purity of the product. This method saves a large number of reagents and solvents used in traditional methods of "introducing protective groups to amino acids" and "removing protective groups from products", reduces the generation of a large amount of chemical waste, saves the time, human resources and energy consumption, and significantly reduces the costs. The cost of peptide synthesis improves the atom economy and step economy of peptide synthesis, effectively achieves energy saving and emission reduction, greatly improves the efficiency of peptide synthesis, and has a broad industrial application prospect.

### DETAILED DESCRIPTION

The content of the present disclosure will be further described in detail below through specific examples. Unless otherwise specified, the raw materials, reagents or devices used in the examples and comparative examples can be obtained from conventional commercial sources, or can be obtained through existing technical methods. Unless otherwise stated, assays or testing methods are routine in the art.

### Example 1

A polypeptide of formula 1 was prepared in the example, with a specific process as follows:

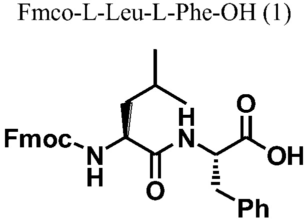

Fmoc-Leu-OH (0.1mmol) and 1-phenylbut-2,3-diene-1-one (0.1 mmol) were taken and dissolved in 1 mL of 1,2-dichloroethane, the mixture was stirred for reaction at a room temperature, and the reaction process was monitored by Thin Layer Chromatography (TLC). After the raw materials were completely consumed, distillation under reduced pressure was performed to remove 1,2-dichloroethane and the mixture was transferred to a system wherein H-Phe-OH (0.2 mmol), N-methyl-N-trimethylsilylacetamide (0.4 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 93%.

Fmoc-Leu-OH (0.1 mmol) and 1-phenylbut-2,3-diene-1-one (0.1 mmol) were taken and dissolved in 1 mL of 1,2-dichloroethane, the mixture was stirred for reaction at a room temperature, and the reaction process was monitored by TLC. After the raw materials were completely consumed, distillation under reduced pressure was performed to remove 1,2-dichloroethane and the mixture was transferred to a system wherein H-Phe-OH (0.2 mmol), hexamethyl disily urea (0.4 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 96%.

Fmoc-Leu-OH (0.1 mmol) and 1-phenylbut-2,3-diene-1-one (0.1 mmol) were taken and dissolved in 1 mL of 1,2-dichloroethane, the mixture was stirred for reaction at a room temperature, and the reaction process was monitored by TLC. After the raw materials were completely consumed, distillation under reduced pressure was performed to remove 1,2-dichloroethane and the mixture was transferred to a system wherein H-Phe-OH (0.2 mmol), trimethylsilimidazole (0.4 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 18%.

Fmoc-Leu-OH (0.1 mmol) and 1-phenylbut-2,3-diene-1-one (0.1 mmol) were taken and dissolved in 1 mL of 1,2-dichloroethane, the mixture was stirred for reaction at a room temperature, and the reaction process was monitored by TLC. After the raw materials were completely consumed, distillation under reduced pressure was performed to remove 1,2-dichloroethane and the mixture was transferred to a system wherein H-Phe-OH (0.2 mmol), N,O-bismethyl trimethylsilylacetamide (0.4 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 94%.

Fmoc-Leu-OH (0.1 mmol) and 1-phenylbut-2,3-diene-1-one (0.1 mmol) were taken and dissolved in 1 mL of 1,2-dichloroethane, the mixture was stirred for reaction at a room temperature, and the reaction process was monitored by TLC. After the raw materials were completely consumed, distillation under reduced pressure was performed to remove 1,2-dichloroethane and the mixture was transferred to a system wherein H-Phe-OH (0.2 mmol), N,O-bismethyl trimethylsilylacetamide (0.4 mmol) and 1mL 1,2-dichloroethane were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and was dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 94%.

Fmoc-Leu-OH (0.1 mmol) and 1-phenylbut-2,3-diene-1-one (0.1 mmol) were taken and dissolved in 1 mL of 1,2-dichloroethane, the mixture was stirred for reaction at a room temperature, and the reaction process was monitored by TLC. After the raw materials were completely consumed, distillation under reduced pressure was performed to remove 1,2-dichloroethane and the mixture was transferred to a system wherein H-Phe-OH (0.2 mmol), N,O-bismethyl trimethylsilylacetamide (0.4 mmol) and 1 mL tetrahydrofuran were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 83%.

Magnetic resonance imaging (NMR) experimental data and mass spectrometry experimental data of the product are as follows:
¹H NMR (400 MHz, DMSO-*d*₆) δ 8.01 (d, J = 7.8 Hz, 1H), 7.89 (d, J = 7.6 Hz, 2H), 7.72 (t, J = 6.1 Hz, 2H), 7.42 (t, J = 7.6 Hz, 3H), 7.32 (t, J = 7.4 Hz, 2H), 7.22 (s, 4H), 7.19 - 7.14 (m, 1H), 4.46 (q, J = 7.4 Hz, 1H), 4.32 (t, J = 8.6 Hz, 1H), 4.29 - 4.18 (m, 2H), 4.07 (td, J = 9.3, 5.3 Hz, 1H), 3.08 (dd, J = 14.0, 5.2 Hz, 1H), 2.94 (dd, J = 13.9, 8.6 Hz, 1H), 1.62 - 1.55 (m, 1H), 1.45 - 1.36 (m, 2H), 0.88 (d, J = 6.6 Hz, 3H), 0.84 (d, J = 6.7 Hz, 3H).
¹³C NMR (101 MHz, DMSO-*d*₆) δ 172.7, 172.2, 155.8, 143.9, 143.7, 140.7, 137.4, 129.1, 128.1, 127.6, 127.0, 126.33, 125.3, 120.1, 65.5, 53.2, 53.0, 46.7, 40.7, 36.7, 24.1, 23.0, 21.5.

### Example 2

A polypeptide of formula 2 was prepared in this example, with a specific process as follows:

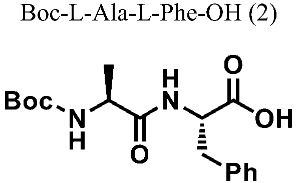

Boc-Ala-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Phe-OH (0.12 mmol), N-trimethylsilylacetamide (0.12 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 94%.

NMR experimental data and mass spectrometry experimental data of the product are as follows:
¹H NMR (400 MHz, DMSO-*d₆*) δ 7.87 (d, J = 7.7 Hz, 1H), 7.28-7.18 (m, J = 15.1, 8.1 Hz, 5H), 6.83 (d, J = 7.3 Hz, 1H), 4.44 (q, J = 7.9 Hz, 1H), 4.06 - 3.86 (m, 1H), 3.06 (dd, J = 13.8, 5.0 Hz, 1H), 2.92 (dd, J = 13.8, 8.4 Hz, 1H), 1.36 (s, 9H), 1.13 (d, J = 7.0 Hz, 3H).
¹³C NMR (101 MHz, DMSO) δ 172.7, 172.5, 154.9, 137.3, 129.2, 128.1, 126.4, 78.0, 53.2, 49.6, 36.7, 28.2, 18.1.

### Example 3

A polypeptide of formula 3 was prepared in this example, with a specific process as follows:

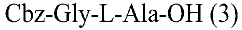

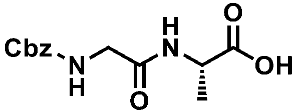

Boc-Gly-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Ala-OH (0.12 mmol), N-trimethylsilylacetamide (0.12 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein the yield was 93%.

NMR experimental data and mass spectrometry experimental data of the product are as follows:
¹H NMR (400 MHz, DMSO-*d*₆) δ 12.64 (s, 1H), 8.15 (d, J = 7.2 Hz, 1H), 7.44 (t, J = 5.9 Hz, 1H), 7.38 - 7.30 (m, 5H), 5.05 (s, 2H), 4.24 (p, J = 7.1 Hz, 1H), 3.71 - 3.63 (m, 2H), 1.28 (d, J = 7.2 Hz, 3H).
¹³C NMR (101 MHz, DMSO-*d*₆) δ 174.1, 168.9, 156.5, 137.1, 128.4, 127.9, 127.7, 65.5, 47.5, 43.3, 17.4.

### Example 4

A polypeptide of formula 4 was prepared in this example, with a specific process as follows:

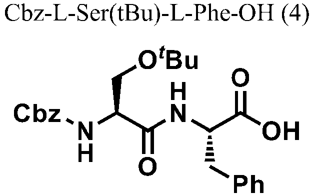

Cbz-Ser(tBu)-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Phe-OH (0.12 mmol), N-trimethylsilylacetamide (0.12 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was purified with column chromatography to obtain a product which was colorless and oily, wherein dr was > 99:1 and the yield was 93%.

NMR experimental data and mass spectrometry experimental data of the product are as follows:
¹H NMR (400 MHz, DMSO-*d₆*) δ 8.01 (d, J = 7.7 Hz, 1H), 7.38 - 7.16 (m, 11H), 5.05 (s, 2H), 4.51 - 4.45 (m, 1H), 4.15 - 4.10 (m, 1H), 3.43 - 3.39 (m, 2H), 3.06 (dd, J = 13.8, 5.3 Hz, 1H), 2.93 (dd, J = 13.8, 8.0 Hz, 1H), 1.09 (s, 9H).
¹³C NMR (101 MHz, DMSO-*d*₆) δ 172.4, 169.6, 155.7, 137.2, 136.9, 129.1, 128.3, 128.1, 127.7, 127.6, 126.4, 72.8, 65.4, 61.8, 55.3, 53.3, 36.8, 27.1.

### Example 5

A polypeptide of formula 5 was prepared in this example, with a specific process as follows:

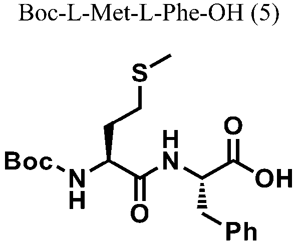

Boc-Met-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Phe-OH (0.12 mmol), N-trimethylsilylacetamide (0.12 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 93%.

NMR experimental data and mass spectrometry experimental data of the product are as follows:
¹H NMR (400 MHz, DMSO-*d*₆) δ 7.94 (d, J = 7.8 Hz, 1H), 7.30 - 7.21 (m, 5H), 6.91 (d, J = 8.2 Hz, 1H), 4.42 - 4.48 (m, 1H), 4.00 (d, J = 7.1 Hz, 1H), 3.07 (dd, J = 13.9, 5.0 Hz, 1H), 2.92 (dd, J = 13.9, 8.7 Hz, 1H), 2.43 - 2.35 (m, 2H), 2.02 (s, 3H), 1.74 (dq, J = 13.7, 7.6, 6.6 Hz, 2H), 1.37 (s, 9H).
¹³C NMR (101 MHz, DMSO-*d₆*) δ 172.8, 171.6, 155.2, 137.3, 129.2, 128.1, 126.4, 78.2, 53.6, 53.2, 36.7, 31.9, 29.6, 28.2, 14.6.

### Example 6

A polypeptide of formula 6 was prepared in this example, with a specific process as follows:

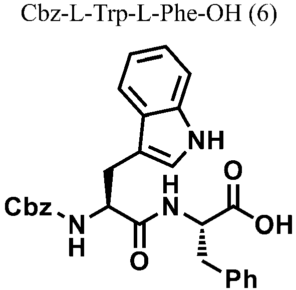

Cbz-Trp-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Phe-OH (0.12 mmol), N-trimethylsilylacetamide (0.12 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 92%.

NMR experimental data and mass spectrometry experimental data of the product are as follows:
¹H NMR (400 MHz, DMSO-*d₆*) δ 10.82 (s, 1H), 8.26 (s, 1H), 7.65 (d, J = 6.7 Hz, 1H), 7.39 - 6.90 (m, 15H), 4.96 (s, 2H), 4.53 (td, J = 8.1, 4.1 Hz, 1H), 4.36 (dt, J = 9.2 4.5 Hz, 1H), 3.11 (t, J = 13.1 Hz, 2H), 3.04 - 2.85 (m, 2H).
¹³C NMR (101 MHz, DMSO-*d₆*) δ 172.8, 171.8, 155.7, 137.4, 137.0, 136.1, 129.2, 128.3, 128.2, 127.7, 127.4, 127.3, 126.4, 123.8, 120.9, 118.5, 118.2, 111.3, 110.2, 65.3, 55.4, 53.4, 36.8, 27.8.

### Example 7

A polypeptide of formula 7 was prepared in this example, with a specific process as follows:

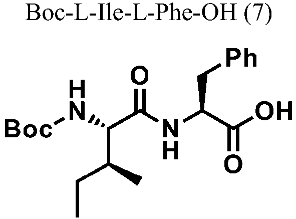

Boc-Ile-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Phe-OH (0.12 mmol), N-trimethylsilylacetamide (0.12 mmol) and 1mL of N,N-dimethylformamide were stirred and mixed in advance, and the reaction process was monitored by TLC. After the reaction was completed, 10 mL of water and 0.2M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 92%.

NMR experimental data and mass spectrometry experimental data of the product are as follows:
¹H NMR (400 MHz, DMSO-*d₆*) δ 12.72 (s, 1H), 8.06 (d, J = 7.9 Hz, 1H), 7.28 - 7.17 (m, 5H), 6.61 (d, J = 9.3 Hz, 1H), 4.50 - 4.44 (m, 1H), 3.82 (t, J = 8.4 Hz, 1H), 3.06 (dd, J = 13.9, 4.9 Hz, 1H), 2.90 (dd, J = 13.9, 9.2 Hz, 1H), 1.61 (q, J = 6.3 Hz, 1H), 1.38 (s, 9H), 1.03 (dq, J = 13.9, 8.1, 7.6 Hz, 1H), 0.88 - 0.82 (m, 1H), 0.77 (t, J = 7.4 Hz, 3H), 0.72 (d, J = 6.7 Hz, 3H).
¹³C NMR (101 MHz, DMSO-*d₆*) δ 172.8, 171.4, 155.2, 137.5, 129.1, 128.1, 126.4, 78.0, 58.7, 53.2, 36.8, 36.7, 28.2, 24.3, 15.2, 10.9.

### Example 8

A polypeptide of formula 8 was prepared in this example, with a specific process as follows:

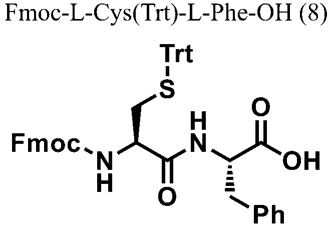

Fmoc-Cys(Trt)-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Phe-OH (0.12 mmol), N-trimethylsilylacetamide (0.12 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and was dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 93%.

NMR experimental data and mass spectrometry experimental data of the product are as follows:
¹H NMR (400 MHz, DMSO-*d₆*) δ 7.89 (d, J = 7.6 Hz, 2H), 7.75 (d, J = 6.5 Hz, 2H), 7.63 (q, J = 8.6 Hz, 1H), 7.41 (t, J = 7.5 Hz, 2H), 7.37 - 7.22 (m, 18H), 7.17 - 7.10 (m, 5H), 4.48 - 4.38 (m, 1H), 4.33 (d, J = 9.5 Hz, 1H), 4.25 (d, J = 5.6 Hz, 2H), 4.04 (dt, J = 19.7, 8.3 Hz, 1H), 3.08 - 3.00 (m, 1H), 2.95 - 2.86 (m, 1H), 2.41 (dq, J = 15.5, 6.9, 4.3 Hz, 2H).
¹³C NMR (101 MHz, DMSO-*d₆*) δ 172.3, 169.6, 155.5, 144.3, 143.6, 140.7, 137.1, 129.1, 128.0, 128.0, 127.6, 127.0, 126.7, 126.3, 125.3, 120.0, 65.9, 53.9, 53.3, 46.6, 36.6, 33.9, 26.3.

### Example 9

A polypeptide of formula 9 was prepared in this example, with a specific process as follows:

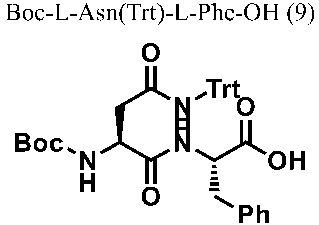

Boc-Asn(Trt)-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Phe-OH (0.12 mmol), N-trimethylsilylacetamide (0.12 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 94%.

NMR experimental data and mass spectrometry experimental data of the product are as follows:
¹H NMR (400 MHz, chloroform-*d*) δ 7.36 (s, 1H), 7.27 - 7.16 (m, 20H), 7.06 (d, J = 6.8 Hz, 2H), 6.16 (d, J = 6.5 Hz, 1H), 4.59 (q, J = 6.3 Hz, 1H), 4.45 (q, J = 5.7 Hz, 1H), 3.01 (dd, J = 13.9, 5.6 Hz, 1H), 2.92 (dd, J = 13.7, 6.0 Hz, 1H), 2.84 (d, J = 10.2 Hz, 1H), 2.60 (dd, J = 14.7, 4.5 Hz, 1H), 1.37 (s, 9H).
¹³C NMR (101 MHz, CDCl₃-*d*) δ 173.6, 171.8, 170.6, 155.9, 144.3, 135.9, 129.4, 128.8, 128.6, 128.0, 127.1, 127.0, 70.9, 53.7, 51.5, 38.1, 37.4, 28.3.

### Example 10

A polypeptide of formula 10 was prepared in this example, with a specific process as follows:

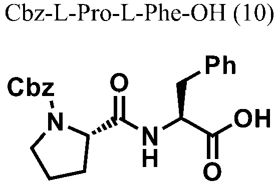

Cbz-Pro-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Phe-OH (0.12 mmol), N-trimethylsilylacetamide (0.12 mmol) and 1 mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 93%.

NMR experimental data and mass spectrometry experimental data of the product are as follows:
¹H NMR (400 MHz, DMSO-*d₆*) δ 8.15 (dd, J = 39.2, 8.0 Hz, 1H), 7.41 - 7.14 (m, 10H), 5.13 - 4.84 (m, 2H), 4.54 - 4.44 (m, 1H), 4.28 - 4.21 (m, 1H), 3.41 - 3.30 (m, 2H), 3.10 - 3.00 (m, 1H), 2.98 - 2.86 (m, 1H), 2.13 - 1.96 (m, 1H), 1.76 - 1.66 (m 3H).
¹³C NMR (101 MHz, DMSO-*d₆*) δ 171.9, 171.8, 171.0, 170.8, 153.1,152.8, 136.6, 136.5, 136.0, 128.2, 128.0, 127.4, 127.2, 127.1, 127.0, 126.7, 126.4, 126.0, 125.4, 125.3, 64.9, 64.7, 58.7, 58.3, 52.4, 52.1, 46.0, 45.4, 38.3, 38.1, 37.9, 35.6, 29.9, 28.6, 22.6, 21.8.

### Example 11

A polypeptide of formula 11 was prepared in this example with a specific process as follows:

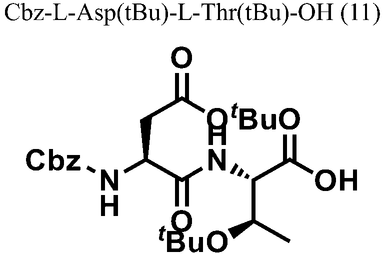

Cbz-Asp(tBu)-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Thr(tBu)-OH (0.12 mmol), N-trimethylsilylacetamide (0.12 mmol) and 1 mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was colorless and oily, wherein dr was > 99:1 and the yield was 94%.

NMR experimental data and mass spectrometry experimental data of the product are as follows:
¹H NMR (400 MHz, DMSO-*d₆*) δ 7.91 - 7.63 (m, 2H), 7.37 - 7.26 (m, 10H), 5.09 - 5.02 (m, 2H), 4.61 - 4.48 (m, 2H), 4.46 - 4.38 (m, 2H), 4.17 - 4.08 (m, 1H), 2.78 - 2.68 (m, 1H), 2.52 - 2.45 (m, 1H), 1.37 (s, 9H), 1.10 (d, J = 6.1 Hz, 3H).
¹³C NMR (101 MHz, DMSO-*d₆*) δ 171.7, 171.2, 169.3, 155.9, 138.4, 136.9, 128.3, 128.1, 127.8, 127.7, 127.4, 127.4,80.1, 74.5, 70.2, 65.6, 56.4, 51.4, 37.4, 27.7, 16.1.

### Example 12

A polypeptide of formula 12 was prepared in this example, with a specific process as follows:

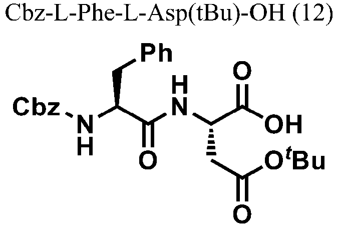

Cbz-Phe-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Asp(tBu)-OH (0.12 mmol), N-trimethylsilylacetamide (0.12 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 93%.

NMR experimental data and mass spectrometry experimental data of the product are as follows:
¹H NMR (400 MHz, chloroform-*d*) δ 7.52 - 7.31 (m, 1H), 7.23 (s, 2H), 7.18 - 7.08 (m, 8H), 5.59 (d, J = 7.8 Hz, 1H), 4.94 (t, J = 8.8 Hz, 2H), 4.71 (dd, J = 8.2, 4.1 Hz, 1H), 4.57 - 4.34 (m, 1H), 3.04 (dd, J = 13.5, 5.7 Hz, 1H), 2.94 (dd, J = 12.9, 7.7 Hz, 1H), 2.87 - 2.79 (m, 1H), 2.67 (dd, J = 17.0, 4.7 Hz, 1H), 1.32 (s, 9H).
¹³C NMR (101 MHz, CDCl₃-*d*) δ 174.0, 171.5, 170.4, 156.3, 136.2, 136.2, 129.5, 128.8, 128.7, 128.3, 128.1, 127.2, 82.3, 67.2, 56.1, 49.0, 38.6, 37.3, 28.1.

### Example 13

A polypeptide of formula 13 was prepared in this example, with a specific process as follows:

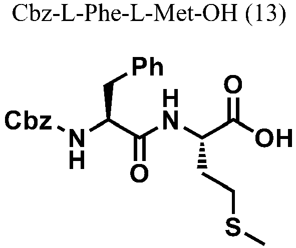

Cbz-Phe-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Met-OH (0.12 mmol), N-trimethylsilylacetamide (0.12 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and was dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 95%.

NMR experimental data and mass spectrometry experimental data of the product are as follows:
¹H NMR (400 MHz, DMSO-*d₆*) δ 8.34 (d, J = 8.1 Hz, 1H), 7.53 (d, J = 8.6 Hz, 1H), 7.28 (dt, J = 24.6, 8.9 Hz, 10H), 4.95 (s, 2H), 4.49 - 4.25 (m, 2H), 3.08 - 2.99 (m, 1H), 2.75 (t, J = 12.4 Hz, 1H), 2.06 (s, 3H), 2.02 (s, 1H), 1.96 - 1.77 (m, 1H), 1.38 (s, 1H), 1.20 (q, J = 10.7, 6.9 Hz, 1H).
¹³C NMR (101 MHz, DMSO-*d₆*) δ 173.3, 171.9, 155.9, 138.2, 137.1, 129.3, 128.4, 128.1, 127.7, 127.5, 126.3, 65.2, 56.1, 51.0, 37.4, 30.8, 29.7, 14.7.

### Example 14

A polypeptide of formula 14 was prepared in this example, with a specific process as follows:

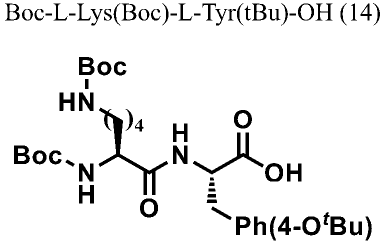

Boc-Lys(Boc)-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Tyr(tBu)-OH (0.12 mmol), N-trimethylsilylacetamide (0.12 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 93%.

NMR experimental data and mass spectrometry experimental data of the product are as follows:
¹H NMR (400 MHz, DMSO-*d₆*) δ 7.87 (d, J = 8.0 Hz, 1H), 7.12 (d, J = 8.4 Hz, 2H), 6.85 (d, J = 8.3 Hz, 2H), 6.77 - 6.67 (m, 2H), 4.42 (td, J = 8.4, 5.0 Hz, 1H), 3.86 (d, J = 8.0 Hz, 1H), 3.01 (dd, J = 14.0, 5.0 Hz, 1H), 2.95 - 2.86 (m, 2H), 2.84 (d, J = 9.3 Hz, 1H), 1.38 (s, 18H), 1.26 (s, 9H).
¹³C NMR (101 MHz, DMSO-*d₆*) δ 172.9, 172.1, 155.6, 155.2, 153.6, 131.9, 129.7, 123.4, 78.0, 77.6, 77.3, 54.4, 53.1, 36.1, 31.7, 29.2, 28.6, 28.3, 28.2, 22.8.

### Example 15

A polypeptide of formula 15 was prepared in this example, with a specific process as follows:

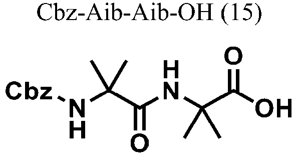

Cbz-Aib-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Aib-OH (0.12 mmol), N-trimethylsilylacetamide (0.12 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein the yield was 88%.

NMR experimental data and mass spectrometry experimental data of the product are as follows:
¹H NMR (400 MHz, DMSO-*d₆*) δ 7.45 (s, 1H), 7.35 (d, J = 4.3 Hz, 4H), 7.31 (dd, J = 5.0, 3.6 Hz, 1H), 7.27 (s, 1H), 5.02 (s, 2H), 1.34 (s, 12H).
¹³C NMR (101 MHz, DMSO-*d₆*) δ 175.8, 173.3, 154.6, 137.1, 128.2, 127.6, 127.5, 65.0, 56.0, 55.1, 25.0, 24.4.

### Example 16

A polypeptide of formula 16 was prepared in this example, with a specific process as follows:

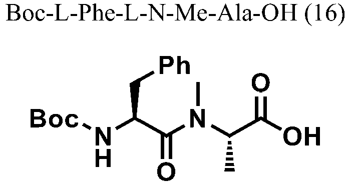

Boc-Phe-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-N-Me-Ala-OH (0.12 mmol), N-trimethylsilylacetamide (0.12 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 95%.

NMR experimental data and mass spectrometry experimental data of the product are as follows:
¹H NMR (400 MHz, DMSO-*d₆*) δ 7.02-6.95 (m, J = 29.2 Hz, 5H), 6.76 (d, J = 8.6 Hz, 1H), 4.73 - 4.46 (m, 1H), 4.40 - 4.13 (m, 1H), 2.67 (d, J = 11.3 Hz, 3H), 2.34 (d, J = 67.9 Hz, 1H), 1.05 (s, 9H), 0.89 (d, J = 35.5 Hz, 3H).
¹³C NMR (101 MHz, DMSO) δ 172.9, 171.6, 155.2, 137.7, 129.4, 128.1, 126.3, 78.1, 52.3, 52.1, 36.8, 31.2, 28.2, 14.1.

### Example 17

A polypeptide of formula 17 was prepared in this example, with a specific process as follows:

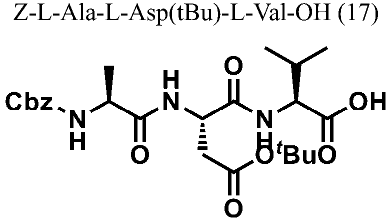

Cbz-Ala-Asp(tBu)-OH (0.1 mmol) and 1-phenylbut-2,3-diene-1-one (0.1 mmol) were taken and dissolved in 1 mL of 1,2-dichloroethane, the mixture was stirred for reaction at a room temperature, and the reaction process was monitored by TLC. After the raw materials were completely consumed, distillation under reduced pressure was performed to remove 1,2-dichloroethane and the mixture was transferred to a system wherein H-Val-OH (0.2 mmol), N,O-bistrimethylsilylacetamide (0.4 mmol) and 1mL of acetonitrile were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 95%.

Cbz-Ala-Asp(tBu)-OH (0.1 mmol) and 1-phenylbut-2,3-diene-1-one (0.1 mmol) were taken and dissolved in 1 mL of 1,2-dichloroethane, the mixture was stirred for reaction at a room temperature, and the reaction process was monitored by TLC. After the raw materials were completely consumed, distillation under reduced pressure was performed to remove 1,2-dichloroethane and the mixture was transferred to a system wherein H-Val-OH (0.2 mmol), hexamethyl disily urea (0.4 mmol) and 1mL of acetonitrile were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 94%.

Cbz-Ala-Asp(tBu)-OH (0.1 mmol) and 1-phenylbut-2,3-diene-1-one (0.1 mmol) were taken and dissolved in 1 mL of 1,2-dichloroethane, the mixture was stirred for reaction at a room temperature, and the reaction process was monitored by TLC. After the raw materials were completely consumed, distillation under reduced pressure was performed to remove 1,2-dichloroethane and the mixture was transferred to a system wherein H-Val-OH (0.2 mmol), N-methyl-N-trimethylsilylacetamide (0.4 mmol) and 1mL of acetonitrile were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 95.7:4.3 and the yield was 92%.

Cbz-Ala-Asp(tBu)-OH (0.1 mmol) and 1-phenylbut-2,3-diene-1-one (0.1 mmol) were taken and dissolved in 1 mL of 1,2-dichloroethane, the mixture was stirred for reaction at a room temperature, and the reaction process was monitored by TLC. After the raw materials were completely consumed, distillation under reduced pressure was performed to remove 1,2-dichloroethane and the mixture was transferred to a system wherein H-Val-OH (0.2 mmol), N,O-bistrimethylsilylacetamide (0.4 mmol) and 1mL of N,N-dimethylformamide were stirred and mixed in advance, and the reaction process was monitored by TLC. After the reaction was completed, 10 mL of water and 0.2M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 97:3 and the yield was 91%.

Cbz-Ala-Asp(tBu)-OH (0.1 mmol) and 1-phenylbut-2,3-diene-1-one (0.1 mmol) were taken and dissolved in 1 mL of 1,2-dichloroethane, the mixture was stirred for reaction at a room temperature, and the reaction process was monitored by TLC. After the raw materials were completely consumed, distillation under reduced pressure was performed to remove 1,2-dichloroethane and the mixture was transferred to a system wherein H-Val-OH (0.12 mmol), N,O-bistrimethylsilylacetamide (0.12 mmol) and 1mL of 1,2-dichloroethane were stirred and mixed in advance, and the reaction process was monitored by TLC. After the reaction was completed, 10 mL of water and 0.2M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 95%.

NMR experimental data and mass spectrometry experimental data of the product are as follows:
¹H NMR (400 MHz, DMSO-*d₆*) δ 8.28 (d, J = 8.1 Hz, 1H), 7.62 (d, J = 8.6 Hz, 1H), 7.52 (d, J = 7.3 Hz, 1H), 7.43 - 7.27 (m, 5H), 5.11 - 4.94 (m, 2H), 4.63 (q, J = 7.4 Hz, 1H), 4.14 (dd, J = 8.6, 5.5 Hz, 1H), 4.05 (q, J = 7.2 Hz, 1H), 2.70 (dd, J = 16.1, 5.9 Hz, 1H), 2.47 (d, J = 7.9 Hz, 1H), 2.05 (q, J = 6.7 Hz, 1H), 1.37 (s, 9H), 1.21 (d, J = 7.1 Hz, 3H), 0.86 (d, J = 6.8 Hz, 6H).
¹³C NMR (101 MHz, DMSO-*d₆*) δ 173.1, 170.7, 169.7, 156.2, 137.4, 128.8, 128.3, 128.2, 80.6, 65.9, 57.6, 50.6, 49.8, 37.4, 30.4, 28.1, 19.5, 18.6, 18.2.

### Example 18

A polypeptide of formula 18 was prepared in this example, with a specific process as follows:

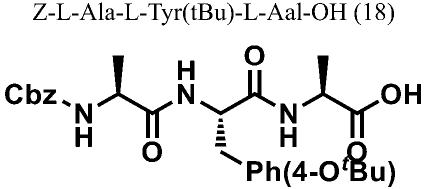

Cbz-Ala-Tyr(tBu)-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Ala-OH (0.12 mmol), N-trimethylsilylacetamide (0.12 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 95%.

NMR experimental data and mass spectrometry experimental data of the product are as follows:
¹H NMR (400 MHz, DMSO-*d₆*) δ 8.24 (d, J = 7.1 Hz, 1H), 7.89 (d, J = 8.5 Hz, 1H), 7.42 (d, J = 7.3 Hz, 1H), 7.38 - 7.26 (m, 5H), 7.16 (d, J = 8.3 Hz, 2H), 6.83 (d, J = 8.2 Hz, 2H), 5.08 - 4.97 (m, 2H), 4.55 (dt, J = 8.5, 4.4 Hz, 1H), 4.24 (p, J = 7.1 Hz, 1H), 4.00 (q, J = 7.0 Hz, 1H), 3.04 (dd, J = 13.8, 3.6 Hz, 1H), 2.78 (dd, J = 13.7, 9.7 Hz, 1H), 1.31 (d, J = 7.2 Hz, 3H), 1.25 (s, 9H), 1.11 (d, J = 7.1 Hz, 3H).
¹³C NMR (101 MHz, DMSO-*d₆*) δ 174.0, 172.2, 170.9, 155.7, 153.4, 137.0, 132.4, 129.9, 128.4, 127.9, 127.8, 123.4, 77.6, 65.5, 53.4, 50.5, 47.7, 36.9, 28.6, 18.2, 17.2.

### Example 19

A polypeptide of formula 19 was prepared in this example, with a specific process as follows:

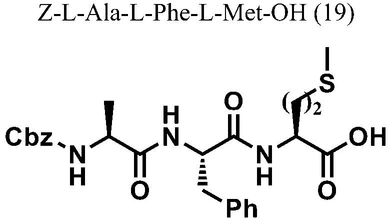

Cbz-Ala-Phe-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Met-OH (0.12 mmol), N-trimethylsilylacetamide (0.12 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and was dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 92%.

NMR experimental data and mass spectrometry experimental data of the product are as follows:
¹H NMR (400 MHz, DMSO-*d₆*) δ 8.19 (d, J = 7.3 Hz, 1H), 7.95 (d, J = 7.7 Hz, 1H), 7.44 (d, J = 7.0 Hz, 1H), 7.41 - 7.13 (m, 10H), 5.11 - 4.94 (m, 2H), 4.61 - 4.52 (m, 1H), 4.42 - 4.35 (m, 1H), 4.06 - 3.99 (m, 1H), 3.15 - 3.04 (m, 1H), 2.92 - 2.80 (m, 1H), 2.53 - 2.43 (m, 2H), 2.05 (s, 3H), 2.03 - 1.96 (m, 1H), 1.94 - 1.86 (m, 1H), 1.14 (d, J = 7.1 Hz, 3H).
¹³C NMR (101 MHz, DMSO-*d₆*) δ 173.0, 172.3, 170.9, 154.9, 137.5, 129.3, 127.9, 126.2,78.1, 53.2, 50.9, 49.9, 37.5, 30.8, 29.6, 28.2, 18.1, 14.6.

### Example 20

A polypeptide of formula 20 was prepared in this example, with a specific process as follows:

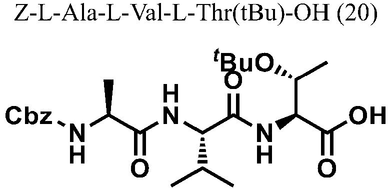

Cbz-Ala-Val-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Thr(tBu)-OH (0.2 mmol), N-trimethylsilylacetamide (0.2 mmol) and 1mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 92%.

NMR experimental data and mass spectrometry experimental data of the product are as follows:
¹H NMR (400 MHz, chloroform-*d*) δ 7.48 - 7.35 (m, 1H), 7.36 - 7.28 (m, 5H), 7.07 - 7.02 (m, 1H), 5.99 - 5.83 (m, 1H), 5.15 - 5.01 (m, 2H), 4.48 (d, J = 7.6 Hz, 1H), 4.45 - 4.31 (m, 2H), 4.27 - 4.20 (m, 1H), 2.13 - 1.99 (m, 1H), 1.33 (d, J = 6.8 Hz, 3H), 1.17 (s, 9H), 1.10 (d, J = 6.0 Hz, 3H), 0.98 - 0.86 (m, 6H).
¹³C NMR (101 MHz, CDCl₃-*d*) δ 173.1, 172.7, 172.0, 156.2, 136.5, 128.7, 128.3, 128.2, 75.5, 67.1, 67.0, 58.8, 57.8, 50.6, 31.5, 28.4, 19.8, 19.1, 19.0, 18.3.

### Example 21

A polypeptide of formula 21 was prepared in this example, with a specific process as follows:

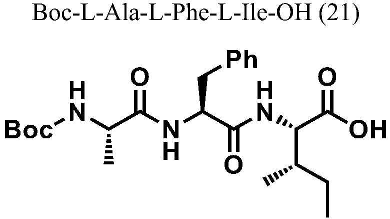

Boc-Ala-Phe-OH (0.1 mmol) and 1-phenylbut-2,3-diene-1-one (0.1 mmol) were taken and dissolved in 1 mL of 1,2-dichloromethane, the mixture was stirred for reaction at a room temperature, and the reaction process was monitored by TLC. After the raw materials were completely consumed, distillation under reduced pressure was performed to remove 1,2-dichloromethane and the mixture was transferred to a system wherein H-Ile-OH (0.12 mmol), N-trimethylsilylacetamide (0.12 mmol) and 1 mL of N,N-dimethylformamide were stirred and mixed in advance, and the reaction process was monitored by TLC. After the reaction was completed, 10 mL of water and 0.2 M HCl were added, an aqueous phase was extracted twice with ethyl acetate, organic phases were combined, the mixture was washed once with water, the organic phase was washed once with saturated brine and dried with anhydrous sodium sulfate, distillation under reduced pressure was performed to remove the solvent, and the mixture was recrystallized with diethyl ether. The mixture was filtered to collect a solid to obtain a pure product which was a white solid, wherein dr was > 99:1 and the yield was 93%.

NMR experimental data and mass spectrometry experimental data of the product are as follows:
¹H NMR (400 MHz, DMSO-*d₆*) δ 8.04 (s, 1H), 7.73 (d, J = 6.1 Hz, 1H), 7.20 (d, J = 14.5 Hz, 5H), 6.96 - 6.62 (m, 1H), 4.63 (s, 1H), 4.20 (s, 1H), 3.90 (s, 1H), 3.03 (d, J = 12.5 Hz, 1H), 2.90 - 2.73 (m, 1H), 1.77 (s, 1H), 1.36 (s, 9H), 1.26 - 1.16 (m, 2H), 1.09 (d, J = 5.4 Hz, 3H), 0.85 (s, 6H).
¹³C NMR (101 MHz, DMSO-*d₆*) δ 172.7, 172.4, 170.8, 154.9, 137.4, 129.3, 127.9, 126.1, 78.1, 56.3, 53.1, 49.9, 37.5, 36.5, 28.1, 24.6, 18.1, 15.5, 11.2.

The above examples are preferred examples of the present disclosure, but the examples of the present disclosure are not limited to the above examples. Any other alternations, modifications, substitutions, combinations, and simplifications made without departing from the spirit and principles of the present disclosure should be equivalent substitutions, and are all included in the protection scope of the present disclosure.

## Claims

1. A method for preparing an amide and/or a polypeptide, comprising the following steps: 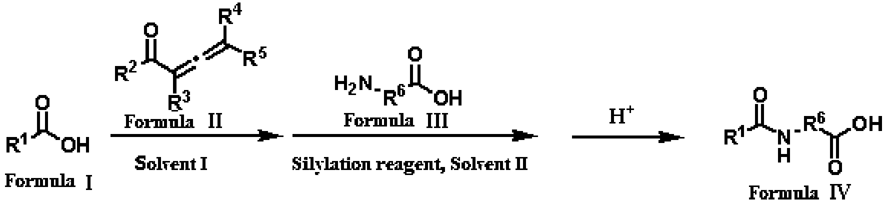
reacting a compound of formula I with a compound of formula II in a solvent I, and carrying out a nucleophilic substitution reaction with a compound of formula III and a silylation reagent in a solvent II, and then obtaining a compound of formula IV after acidification;
wherein R¹ comprises alkyl, alkenyl, alkynyl, cycloalkyl, heterocycloalkyl, aryl, heteroaryl, an amino acid residual body lacking C-terminal carboxyl, an amino acid derivative residual body or a polypeptide fragment lacking C-terminal carboxyl; R² comprises one of aryl, substituted aryl, heteroaryl, and substituted heteroaryl; R³, R⁴ and R⁵ each independently comprise one of H, C₁-C₁₈ hydrocarbonyl, substituted C₁-C₁₈ hydrocarbonyl, C₁-C₁₆ acyl, cyano, and C₁-C₁₆ hydrocarbonyl-carbonyl; R⁶ comprises alkyl, cycloalkyl, heterocycloalkyl, aryl, heteroaryl, an amino acid residue or a polypeptide fragment.

2. A method for preparing an amide and/or a polypeptide, comprising the following steps: 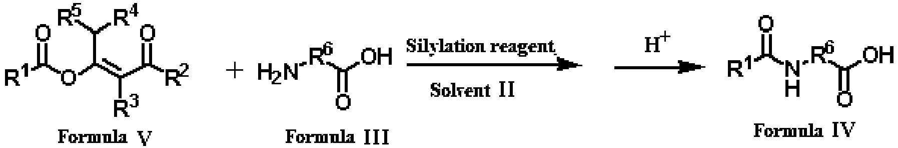
dissolving a compound of formula V, a compound of formula III and a silylation reagent in a solvent II to perform a nucleophilic substitution reaction, and then preparing a compound of formula IV after acidification;
wherein R¹, R², R³, R⁴, R⁵ and R⁶ are defined as in claim 1.

3. The method for preparing an amide and/or a polypeptide according to claim 2, wherein the method for preparing the compound of formula V comprises the following steps: 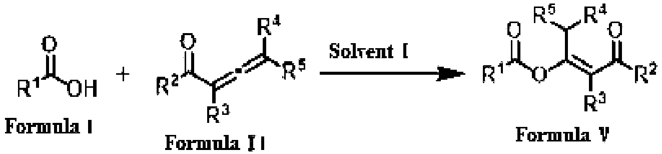
dissolving a compound of formula I and a compound of formula II in the solvent I to obtain a mixture, and reacting the mixture under stirring to prepare a compound of formula V;
wherein R¹, R², R³, R⁴ and R⁵ are defined as in claim 1.

4. A method for preparing an amide and/or a polypeptide, comprising the following steps: 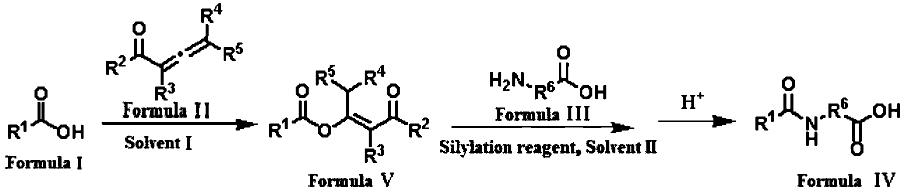
S1: dissolving a compound of formula I and a compound of formula II in a solvent I to obtain a mixture, and reacting the mixture under stirring to prepare a compound of formula V;
S2: dissolving the compound of formula V, the compound of formula III, and the silylation reagent in a solvent II to perform a nucleophilic substitution reaction, and then preparing a compound of formula IV after acidification;
wherein R¹, R², R³, R⁴, R⁵ and R⁶ are defined as in claim 1.

5. The method for preparing an amide and/or a polypeptide according to any one of claims 1, 3 or 4, wherein when the solvent II is different from the solvent I, the method for preparing an amide and/or a polypeptide further comprises removing the solvent I after the reaction and then performing the nucleophilic substitution reaction.

6. The method for preparing an amide and/or a polypeptide according to any one of claims 1, 2 or 4, wherein the silylation reagent comprises at least one of N-trimethylsilylacetamide, N-methyl-N-trimethylsilylacetamide, N-trimethylsilylpyrrolidone, N-(tert-butyldimethylsilyl)-N-methyltrifluoroacetamide, N,O-bis(tertbutyldimethylsilyl)acetamide, N,O-bistrimethylsilylacetamide, N,O-bis(trimethylsilanyl)trifluoroacetamide, N-methyl-N-(trimethylsilyl)trifluoroacetamide, hexamethyldisilylurea, trimethylsilimidazole, dimethyl dichlorosilane, trimethylchlorosilane and tert-butyldimethylchlorosilane.

7. The method for preparing an amide and/or a polypeptide according to any one of claims 1, 2 or 4, wherein a molar ratio of the silylation reagent to the compound of formula III is (0.5-20): 1.

8. The method for preparing an amide and/or a polypeptide according to any one of claims 1, 2 or 4, wherein the solvent II comprises at least one of acetonitrile, diethyl ether, ethylene glycol dimethyl ether, ethylene glycol diethyl ether, dioxane, dimethyl sulfoxide, N,N-dimethylformamide, N,N-dimethylacetamide, N-methylpyrrolidone, toluene, m-xylene, dichloromethane, 1,2-dichloroethane or chloroform.

9. The method for preparing an amide and/or a polypeptide according to any one of claims 1, 3 or 4, wherein the solvent I comprises at least one of dichloromethane, chloroform, 1,2-dichloroethane, diethyl ether, toluene, acetonitrile, methanol or ethanol.

10. The method for preparing an amide and/or a polypeptide according to any one of claims 1, 3 or 4, wherein a molar ratio of the compound of formula I to the compound of formula II is 1: (1-5).

11. Use of the method for preparing an amide and/or a polypeptide in preparing amides and/or polypeptides.
